# EUROPÄISCHE PATENTSCHRIFT

(11) **EP 0 521 823 B1**
(45) Veröffentlichungstag und Bekanntmachung des Hinweises auf die Patenterteilung: **17.01.1996**
(21) Anmeldenummer: 92810481.9
(22) Anmeldetag: 24.06.1992
(51) Int. Cl.: C07D 333/22

(54) **Phenylthiophenylketone**
Phenylthiophenyl ketones
Phénylthiophényl-cétones

(30) Priorität: 03.07.1991 CH 1963/91
(43) Veröffentlichungstag der Anmeldung: 07.01.1993
(73) Patentinhaber: CIBA-GEIGY AG, CH-4002 Basel (CH)
(72) Erfinder: Leppard, David G., Dr., CH-1723 Marly (CH); Burdeska, Kurt, Dr., CH-4058 Basel (CH)

(56) Entgegenhaltungen:
- FR-A- 1 336 942
- US-A- 3 785 827
- Römpps Chemie-Lexikon, 8. Aufl., Band 6, Seiten 4452-4456, "UV-Spektroskopie"

## Beschreibung

Die vorliegende Anmeldung betrifft neue Phenylthiophenylketone und ihre Verwendung als UV-Absorber in photographischen Materialien, insbesondere solchen mit transparenten Trägern.

Es ist bekannt, in photographischen Materialien UV-Absorber zu verwenden, um den schädigenden Einfluss von UV-Licht auf das Material bei der Belichtung zu vermindern. Ferner tragen UV-Absorber bekanntlich dazu bei, die bei der Herstellung photographischer Materialien auftretenden Verblitzungen weitgehend unwirksam zu machen.

In der GB-B-973,919 sind Phenylthiophenylketone der Formel 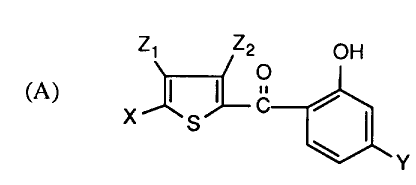 beschrieben, worin Z₁ und Z₂

Wasserstoff oder Alkylreste sind, X Wasserstoff, ein Alkylrest, Phenyl oder Halogenphenyl und Y Hydroxyl oder eine verätherte Hydroxygruppe ist. Ihre Anwendung in Filterschichten photographischer Materialien wird erwähnt.

Es hat sich jedoch gezeigt, dass die in dem genannten Dokument offenbarten Verbindungen als UV-Absorber in photographischen Materialien zu wenig wirksam sind und auf Grund ihrer schlechten Löslichkeit in dafür üblichen Lösungsmitteln nur zu einem geringen Teil in photographische Schichten eingearbeitet werden können.

Nun wurden neue Verbindungen des obengenannten Typs gefunden, die sich leichter in photographische Schichten einbringen lassen und eine erhöhte Aktivität als UV-Absorber zeigen.

Gegenstand vorliegender Erfindung sind somit Verbindungen der Formel 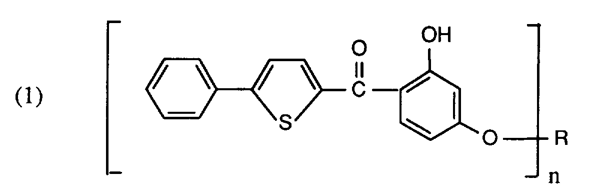 worin n 1, 2, 3 oder 4 ist, und
R, wenn n 1 ist, Alkyl mit 1 bis 18 Kohlenstoffatomen substituiert mit -CO₂R₂ ist oder R -CH₂CH(OH)CH₂OR₁ ist,
wobei R₁ Wasserstoff, Alkyl mit 1 bis 18 Kohlenstoffatomen, Cyclohexyl, Benzyl, Phenyl, Tolyl, -CO-Alkenyl mit 2 bis 4 Kohlenstoffatomen im Alkenylteil oder Glycidyloxyalkyl mit 3 bis 14 Kohlenstoffatomen im Alkylteil,
R₂ Alkyl mit 1 bis 18 Kohlenstoffatomen, durch Sauerstoff unterbrochenes Alkyl oder Hydroxyalkyl mit je 3 bis 14 Kohlenstoffatomen, Hydroxyalkyl mit 2 bis
18 Kohlenstoffatomen, Glycidyl, Cycloalkyl mit 5 bis 12 Kohlenstoffatomen, Alkenyl mit 3 bis 18 Kohlenstoffatomen, Benzyl, Phenyl, Alkylphenyl mit 1 bis 12 Kohlenstoffatomen im Alkylteil, Furfuryl oder ein Rest der Formel -CH₂CH(OH)CH₂OR₁ ist,
oder
R, wenn n 2 ist, ein Rest der Formel -CH₂CH(OH)CH₂-O-R₁₂-O-CH₂CH(OH)CH₂- oder -(CH₂)ₘCO-O-R₁₄-O-CO(CH₂)ₘ-, worin m 1, 2 oder 3 ist, ist,
wobei R₁₂ Alkylen mit 2 bis 10 Kohlenstoffatomen, durch Sauerstoff unterbrochenes Alkylen mit 3 bis 14 Kohlenstoffatomen, Phenylen oder ein Rest der Formel 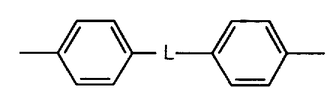 ist, worin L -O-, -S-, -SO₂-, -CH₂- oder -C(CH₃)₂- ist,
und
R₁₄ Alkylen mit 2 bis 10 Kohlenstoffatomen oder durch Sauerstoff unterbrochenes Alkylen mit 4 bis 30 Kohlenstoffatomen ist, oder
R, wenn n 3 ist, ein Rest der Formel 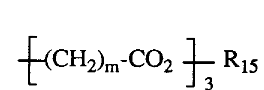 ist, worin m die angegebene Bedeutung hat, und
R₁₅ ein Alkantriyl mit 3 bis 12 Kohlenstoffatomen ist, oder
R, wenn n 4 ist, ein Rest der Formel 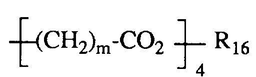 ist, worin m die angegebene Bedeutung hat, und
R₁₆ ein Alkantetrayl mit 4 bis 12 Kohlenstoffatomen ist.

Weitere Gegenstände der vorliegenden Erfindung sind Verfahren zur Herstellung der Verbindungen der Formel (1) sowie photographisches Material, das eine Schicht mit einer solchen Verbindung enthält.

In den oben aufgezählten Definitionen bedeuten Alkylreste mit 1 bis 18 Kohlenstoffatomen beispielsweise Methyl, Ethyl, Propyl, Butyl, Pentyl, Hexyl, Heptyl, Octyl, Nonyl, Decyl, Undecyl, Dodecyl, Tetradecyl, Hexadecyl und Octadecyl sowie entsprechende verzweigte Isomere.

Beispiele für Alkylenreste lassen sich von den genannten Alkylresten ableiten.

Als Beispiele für die erfindungsgemäss verwendeten substituierten Alkylreste mit 1 bis 18 Kohlenstoffatomen seien genannt: -CH₂CH(OH)CH₂OC₄H₉, -CH₂CH(OH)CH₂OC₁₂H₂₅, -CH₂CH(OH)CH₂OC₆H₅ -CH₂COOC₂H_{5,} -CH₂COOC₈H₁₇,-CH₂CH₂COOCH₃, -CH₂CH₂COOC₄H₉ oder -CH₂CH₂COOC₁₂H₂₅.

Beispiele für Alkylreste, die durch Sauerstoff unterbrochen sind, stellen die folgenden Gruppen dar: 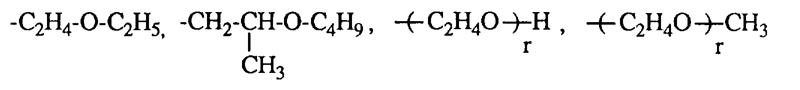 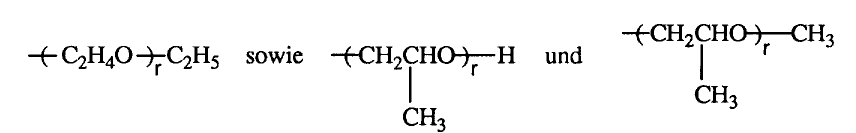 (mit r = 2-15).

Cycloalkylreste, die erfindungsgemäss verwendbar sind, umfassen beispielsweise Cyclopentyl, Cyclohexyl, Cyclooctyl, Cycloundecyl und Cyclododecyl.

Bevorzugte Verbindungen der Formel (1) sind solche, worin
R, wenn n 1 ist, mit -CO₂R₂ substituiertes Alkyl mit 1 bis 15 Kohlenstoffatomen oder -CH₂CH(OH)CH₂OR₁ ist,
wobei R₁ Wasserstoff, Alkyl mit 1 bis 18 Kohlenstoffatomen, Cyclohexyl oder Phenyl, R₂ Alkyl mit 1 bis 12 Kohlenstoffatomen, durch Sauerstoff unterbrochenes Alkyl oder Hydroxyalkyl mit je 3 bis 14 Kohlenstoffatomen, Hydroxyalkyl mit 2 bis
12 Kohlenstoffatomen, Alkenyl mit 3 bis 18 Kohlenstoffatomen, Cyclohexyl, Glycidyl, Benzyl, Alkylphenyl mit 1 bis 12 Kohlenstoffatomen im Alkylteil, Phenyl oder Furfuryl ist,
oder
R, wenn n 2 ist, ein Rest der Formel -CH₂CH(OH)CH₂-O-R₁₂-O-CH₂CH(OH)CH₂- oder -(CH₂)ₘCO-O-R₁₄-O-CO(CH₂)ₘ- ist, worin m 1 oder 2 ist,
wobei R₁₂ Alkylen mit 2 bis 10 Kohlenstoffatomen und
R₁₄ Alkylen mit 2 bis 10 Kohlenstoffatomen oder durch Sauerstoff unterbrochenes Alkylen mit 4 bis 20 Kohlenstoffatomen ist.

Hiervon sind besonders die Verbindungen geeignet, worin
R, wenn n 1 ist, mit -CO₂R₂ substituiertes Alkyl mit 1 bis 3 Kohlenstoffatomen oder -CH₂CH(OH)CH₂OR₁ ist,
wobei R₁ Wasserstoff, Alkyl mit 1 bis 18 Kohlenstoffatomen, Cyclohexyl oder Phenyl, R₂ Alkyl mit 1 bis 8 Kohlenstoffatomen, durch Sauerstoff unterbrochenes Alkyl mit 3 bis 12 Kohlenstoffatomen, Vinyl, Allyl, Cyclohexyl, Benzyl oder Glycidyl und
R, wenn n 2 ist, ein Rest der Formel -(CH₂)₂CO-O-R₁₄-O-CO(CH₂)₂- ist,
wobei R₁₄ Alkylen mit 2 bis 4 Kohlenstoffatomen oder durch Sauerstoff unterbrochenes Alkylen mit 4 bis 12 Kohlenstoffatomen ist,
und insbesondere solche,
worin n 1 ist, und R ein Rest der Formel -CH₂CH(OH)CH₂OR₁ ist, worin R₁ Wasserstoff oder Alkyl mit 1 bis 18 Kohlenstoffatomen, Cyclohexyl oder Phenyl ist.

Eine weitere Gruppe bevorzugter Verbindungen der Formel (1) ist dadurch gekennzeichnet, dass R, wenn n 2 ist, ein Rest der Formel 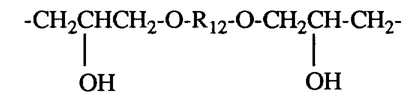 ist, wobei R₁₂ die oben angegebene Bedeutung hat.

Die Verbindungen der Formel (1) können beispielsweise durch Umsetzung der Ausgangsverbindung der Formel 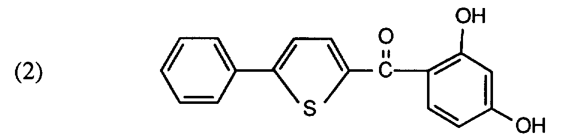 mit einer entsprechenden Halogenverbindung der Formel

(3) R-X

worin R die angegebene Bedeutung hat und X Halogen, insbesondere Chlor oder Brom, ist, hergestellt werden. Dazu löst man die Verbindungen der Formeln (2) und (3) in einem geeigneten Lösungsmittel, z.B. einem Keton, einem Alkohol oder aromatischen Lösungsmittel, und erhitzt die Lösung bei erhöhter Temperatur in Gegenwart einer Base. Diese Verknüpfungsreaktion lässt sich auch durchführen, wenn die Komponenten in dem gewählten Lösungsmittel nicht gut löslich sind und in Form einer Dispersion vorliegen.

Die Ausgangsprodukte der Formel (2) sind beispielsweise nach dem in der genannten GB-B-973,919 geschilderten Verfahren erhältlich.

Für die Herstellung solcher Verbindungen der Formel (1), worin n 1, R
-CH₂CH(OH)CH₂OR₁ und R₁ Wasserstoff, C₁-C₁₈-Alkyl, Cyclohexyl oder Phenyl ist, werden entsprechend Epoxidverbindungen der Formel 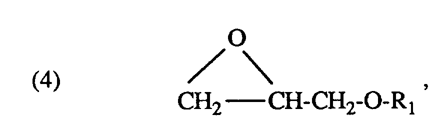 worin R₁ die angegebene Bedeutung hat, eingesetzt.

Für die Herstellung solcher Verbindungen der Formel (1), worin n 2 und R 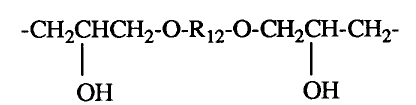 ist, werden entsprechend zwei Epoxidgruppen enthaltende Komponenten der Formel 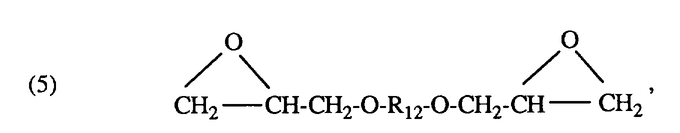 worin R₁₂ die angegebene Bedeutung hat, eingesetzt.

Die erfindungsgemässen Verbindungen der Formel (1) absorbieren wirksam Strahlung im Bereich von 300 bis 400 nm und sind somit als UV-Absorber geeignet. Insbesondere lassen sie sich als UV-Absorber in photographischen Materialien anwenden, da sie die folgenden Kriterien gut erfüllen:
(1) sie beeinträchtigen nicht die sensitometrischen Eigenschaften der Materialien, in denen sie vorliegen,
(2) sie verändern sich nicht weder bei der Belichtung noch bei der Verarbeitung sowie der anschliessenden Lagerung der Materialien;
(3) sie sind stabil gegen UV-Strahlung, Wärme und Feuchtigkeit.

Auf Grund der leicht gelblichen Eigenfarbe der erfindungsgemässen Verbindungen sind sie weniger in photographischen Papiermaterialien als vielmehr in Filmmaterialien verwendbar. Solche Materialien können auf üblichen transparenten Trägern, wie sie beispielsweise aus RESEARCH DISCLOSURE, November 1989, Seite 879, bekannt sind, lichtempfindliche Schichten, Antihaloschichten, Zwischenschichten sowie Sperr- und Schutzschichten in allen bekannten Abfolgen aufweisen. Beispiele von Materialien dieser Art sind im genannten RESEARCH DISCLOSURE auf Seite 872 beschrieben.

Schichten, welche mindestens eine der erfindungsgemässen Verbindungen enthalten, können an jeder Stelle des Materials eingelagert werden. Solche Schichten können (das Bindemittel ausgenommen) frei von weiteren Komponenten sein. Sie können aber auch photographisch wirksame Substanzen wie z.B. Silberhalogenid und/oder Farbkuppler enthalten. Vorzugsweise ordnet man Bindemittelschichten mit den UV-Absorbern über der obersten Silberhalogenidemulsionsschicht, zwischen zwei Silberhalogenidemulsionsschichten oder auf der Rückseite des Trägers an.

Vorzugsweise liegen die Verbindungen der Formel (1) mit einem Giessgewicht von 5 bis 1500 mg/m, insbesondere 5 bis 700 mg/m in der betreffenden Schicht vor.

Die Verbindungen lassen sich mit Hilfe geeigneter Lösungsmittel, beispielsweise einem gemäss Seite 877 der genannten RESEARCH DISCLOSURE in üblicher Weise in das betreffende Bindemittel einarbeiten, wobei die bekannten, z.B. auf Seite 873 dieses Zitats aufgezählten Bindemittel in Frage kommen können.

Die folgenden Beispiele erläutern die Erfindung weiter.

Beispiel 1: 29,6 g der Verbindung der Formel (2), 14,3 g Butylglycidylether und 2,6 g Ethyltriphenylphosphoniumbromid werden 1 Stunde bei 130°C unter Rühren erhitzt. Das Reaktionsgemisch wird in 100 ml Toluol aufgelöst, mit Wasser gewaschen und getrocknet. Nach Entfernen des Lösungsmittels unter erniedrigtem Druck erhält man einen Rückstand, der aus Ethanol umkristallisiert wird. Man isoliert so die Verbindung der Formel 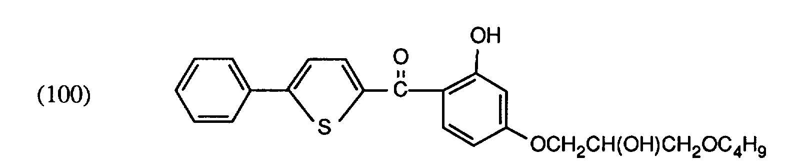 in einer Ausbeute von 73 % (31,0 g) in Form gelber Kristalle mit einem Schmelzpunkt von 72°C;

| | | | | |
|---|---|---|---|---|
| Elementaranalyse: | berechnet | 67,58 % C | 6,14 % H | 7,51 % S |
| | gefunden | 67,43 % C | 6,10 % H | 7,80 % S |

Auf analoge Weise, jedoch mit 20,5 g Ethylhexylglycidyläther anstelle von Butylglycidyläther, erhält man die Verbindung der Formel 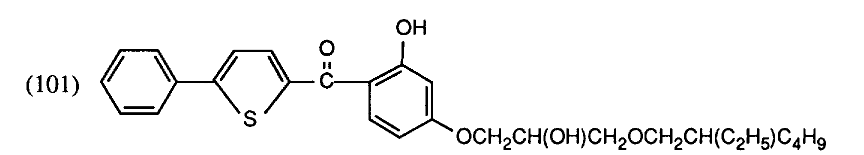 in einer Ausbeute von 71 % (34,2 g)
mit einem Schmelzpunkt von 45°C;

| | | | | |
|---|---|---|---|---|
| Elementaranalyse: | berechnet | 69,68 % C | 7,10 % H | 6,64 % S |
| | gefunden | 69,70 % C | 7,08 % H | 6,63 % S. |

Analog können auch die in der folgenden Tabelle aufgeführten Verbindungen hergestellt werden:

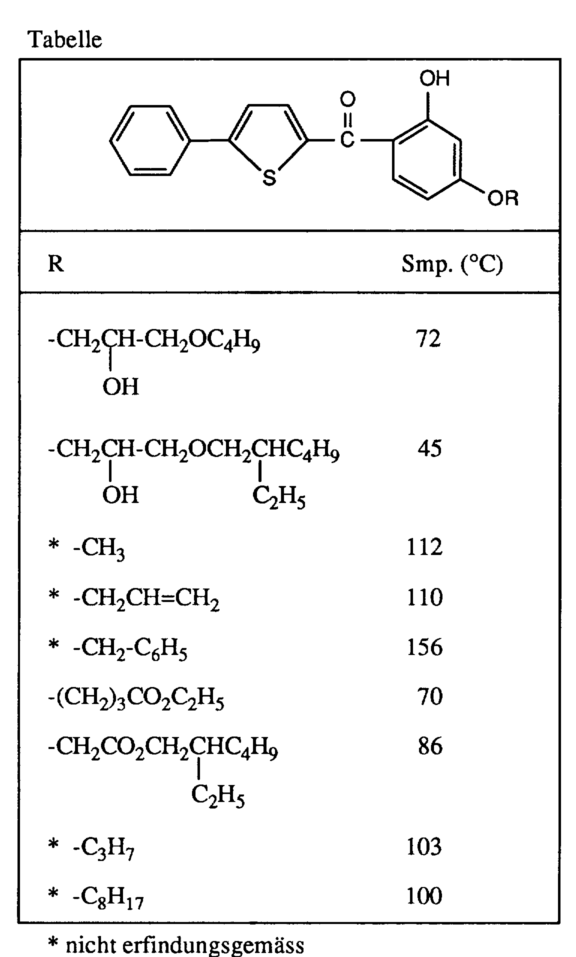

Beispiel 2: 102 mg UV-Absorber werden in 2 ml Ethylacetat gelöst. 1 ml dieser Lösung wird mit 9 ml einer wässrigen Gelatinelösung (enthaltend 27,6 g/l Gelatine und 6,8 g/l 8%ige wässrige Lösung des 4,8-Diisobutylnaphthyl-2-natriumsulfonats) mittels Ultraschall während 3 Minuten emulgiert. 7,5 ml der so erhaltenen Emulsion werden mit 4,5 ml 0,24%iger wässriger Lösung des Natriumsalzes des 2-Hydroxy-4,6-dichlor-1,3,5-triazins versetzt. 8 ml dieser Emulsion werden als Schicht auf einen Polyesterträger (13x18 cm) aufgebracht. Der UV-Absorber liegt damit in einem Giessgewicht von 1,09 g/m vor. Nach einer Trocknungszeit von 7 Tagen bei Raumtemperatur wird das UV-Absorptionsspektrum der Probe aufgenommen. Dieses zeichnet sich durch eine breite Absorptionsbande mit einem Maximum bei 372 nm aus.

## Patentansprüche

1. Verbindung der Formel 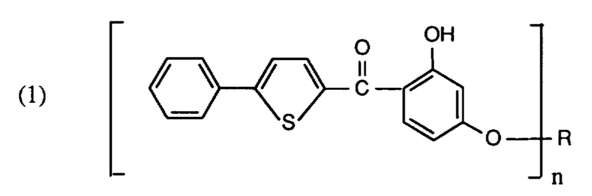
worin n 1, 2, 3 oder 4 ist, und
R, wenn n 1 ist, Alkyl mit 1 bis 18 Kohlenstoffatomen substituiert mit -CO₂R₂ ist oder R -CH₂CH(OH)CH₂OR₁ ist,
wobei R₁ Wasserstoff, Alkyl mit 1 bis 18 Kohlenstoffatomen, Cyclohexyl, Benzyl, Phenyl, Tolyl, -CO-Alkenyl mit 2 bis 4 Kohlenstoffatomen im Alkenylteil oder Glycidyloxyalkyl mit 3 bis 14 Kohlenstoffatomen im Alkylteil,
R₂ Alkyl mit 1 bis 18 Kohlenstoffatomen, durch Sauerstoff unterbrochenes Alkyl oder Hydroxyalkyl mit je 3 bis 14 Kohlenstoffatomen, Hydroxyalkyl mit 2 bis
18 Kohlenstoffatomen, Glycidyl, Cycloalkyl mit 5 bis 12 Kohlenstoffatomen, Alkenyl mit 3 bis 18 Kohlenstoffatomen, Benzyl, Phenyl, Alkylphenyl mit 1 bis 12 Kohlenstoffatomen im Alkylteil, Furfuryl oder ein Rest der Formel -CH₂CH(OH)CH₂OR₁ ist,
oder
R, wenn n 2 ist, ein Rest der Formel -CH₂CH(OH)CH₂-O-R₁₂-O-CH₂CH(OH)CH₂- oder -(CH₂)ₘCO-O-R₁₄-O-CO(CH₂)ₘ-, worin m 1,2 oder 3 ist, ist,
wobei R₁₂ Alkylen mit 2 bis 10 Kohlenstoffatomen, durch Sauerstoff unterbrochenes Alkylen mit 3 bis 14 Kohlenstoffatomen, Phenylen oder ein Rest der Formel 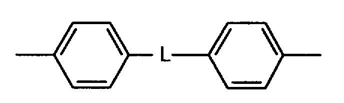 ist, worin L -O-, -S-, -SO₂-, -CH₂- oder -C(CH₃)₂- ist,
und
R₁₄ Alkylen mit 2 bis 10 Kohlenstoffatomen oder durch Sauerstoff unterbrochenes Alkylen mit 4 bis 30 Kohlenstoffatomen ist, oder
R, wenn n 3 ist, ein Rest der Formel 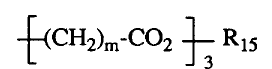 ist, worin m die angegebene Bedeutung hat, und
R₁₅ ein Alkantriyl mit 3 bis 12 Kohlenstoffatomen ist, oder
R, wenn n 4 ist, ein Rest der Formel 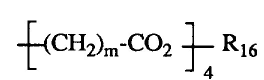 ist, worin m die angegebene Bedeutung hat, und
R₁₆ ein Alkantetrayl mit 4 bis 12 Kohlenstoffatomen ist.

2. Verbindungen nach Anspruch 1, worin
R, wenn n 1 ist, mit -CO₂R₂ substituiertes Alkyl mit 1 bis 15 Kohlenstoffatomen oder -CH₂CH(OH)CH₂OR₁ ist,
wobei R₁ Wasserstoff, Alkyl mit 1 bis 18 Kohlenstoffatomen, Cyclohexyl oder Phenyl, R₂ Alkyl mit 1 bis 12 Kohlenstoffatomen, durch Sauerstoff unterbrochenes Alkyl oder Hydroxyalkyl mit je 3 bis 14 Kohlenstoffatomen, Hydroxyalkyl mit 2 bis
12 Kohlenstoffatomen, Alkenyl mit 3 bis 18 Kohlenstoffatomen, Cyclohexyl, Glycidyl, Benzyl, Alkylphenyl mit 1 bis 12 Kohlenstoffatomen im Alkylteil, Phenyl oder Furfuryl ist,
R, wenn n 2 ist, ein Rest der Formel -CH₂CH(OH)CH₂-O-R₁₂-O-CH₂CH(OH)CH₂- oder -(CH₂)ₘCO-O-R₁₄-O-CO(CH₂)ₘ- ist, worin m 1 oder 2 ist,
wobei R₁₂ Alkylen mit 2 bis 10 Kohlenstoffatomen und
R₁₄ Alkylen mit 2 bis 10 Kohlenstoffatomen oder durch Sauerstoff unterbrochenes Alkylen mit 4 bis 20 Kohlenstoffatomen ist.

3. Verbindungen nach Anspruch 2, worin
R, wenn n 1 ist, mit -CO₂R₂ substituiertes Alkyl mit 1 bis 3 Kohlenstoffatomen oder -CH₂CH(OH)CH₂OR₁ ist,
wobei R₁ Wasserstoff, Alkyl mit 1 bis 18 Kohlenstoffatomen, Cyclohexyl oder Phenyl, R₂ Alkyl mit 1 bis 8 Kohlenstoffatomen, durch Sauerstoff unterbrochenes Alkyl mit 3 bis 12 Kohlenstoffatomen, Vinyl, Allyl, Cyclohexyl, Benzyl oder Glycidyl und
R, wenn n 2 ist, ein Rest der Formel -(CH₂)₂CO-O-R₁₄-O-CO(CH₂)₂- ist,
wobei R₁₄ Alkylen mit 2 bis 4 Kohlenstoffatomen oder durch Sauerstoff unterbrochenes Alkylen mit 4 bis 12 Kohlenstoffatomen ist.

4. Verbindungen nach Anspruch 3, worin
n 1 ist und R ein Rest der Formel -CH₂CH(OH)CH₂OR₁ ist, worin R₁ Wasserstoff oder Alkyl mit 1 bis 18 Kohlenstoffatomen, Cyclohexyl oder Phenyl ist.

5. Verbindungen nach Anspruch 1, worin R, wenn n 2 ist, ein Rest der Formel 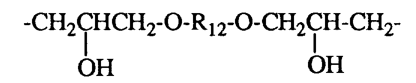 ist, wobei R₁₂ die in Anspruch 1 angegebene Bedeutung hat.

6. Verfahren zur Herstellung der Verbindungen der Formel (1) nach Anspruch 1, dadurch gekennzeichnet, dass man die Verbindung der Formel 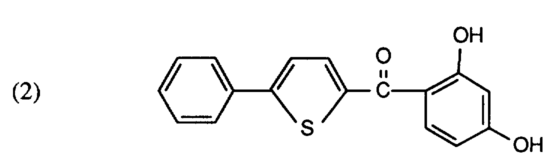 mit Halogenverbindungen der Formel
(3) R-X,
worin R die in Anspruch 1 angegebene Bedeutung hat, und X Halogen, vorzugsweise Chlor oder Brom ist, umsetzt.

7. Verfahren zur Herstellung der Verbindungen der Formel (1) nach Anspruch 4, dadurch gekennzeichnet, dass man die Verbindung der Formel 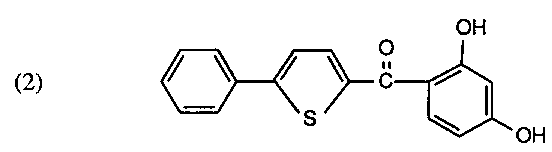 mit einer Epoxidverbindung der Formel 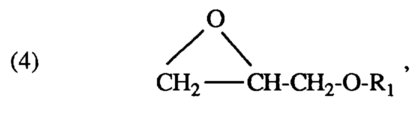 worin R₁ die in Anspruch 4 angegebene Bedeutung hat, umsetzt.

8. Verfahren zur Herstellung der Verbindungen der Formel (1) nach Anspruch 5, dadurch gekennzeichnet, dass man die Verbindung der Formel 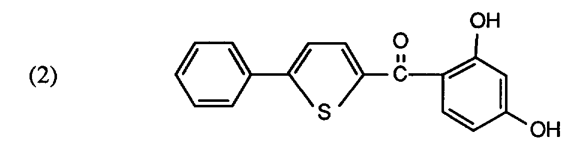 mit einer Epoxidverbindung der Formel 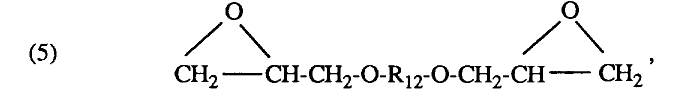 worin R₁₂ die in Anspruch 5 angegebene Bedeutung hat, umsetzt.

9. Photographisches Material enthaltend auf einem transparenten Träger eine oder mehrere Silberhalogenidemulsionschichten, dadurch gekennzeichnet, dass es als zusätzliche Schicht eine solche aufweist, die mindestens eine Verbindung der Formel (1) enthält.

10. Photographisches Material nach Anspruch 9, dadurch gekennzeichnet, dass es die zusätzliche Schicht über der obersten Silberhalogenidemulsionsschicht, zwischen zwei Silberhalogenidemulsionsschichten oder auf der Rückseite des Trägers enthält.

## Claims

1. A compound of formula 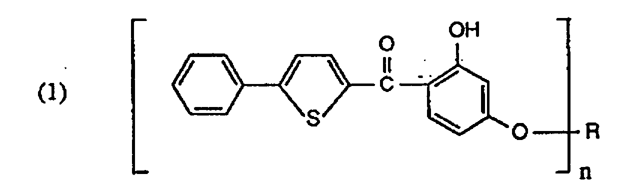 wherein n is 1, 2, 3 or 4, and
R, if n is 1, is alkyl of 1 to 18 carbon atoms which is substituted by -CO₂R₂ or R is -CH₂CH(OH)CH₂OR₁,
and R₁ is hydrogen, alkyl of 1 to 18 carbon atoms, cyclohexyl, benzyl, phenyl, tolyl, -CO-alkenyl containing 2 to 4 carbon atoms in the alkenyl moiety or glycidyloxyalkyl containing 3 to 14 carbon atoms in the alkyl moiety,
R₂ is alkyl of 1 to 18 carbon atoms, alkyl or hydroxyalkyl each of 3 to 14 carbon atoms and interrupted by oxygen, hydroxyalkyl of 2 to 18 carbon atoms, glycidyl, cycloalkyl of 5 to 12 carbon atoms, alkenyl of 3 to 18 carbon atoms, benzyl, phenyl, alkylphenyl containing 1 to 12 carbon atoms in the alkyl moiety, furfuryl or a radical of formula -CH₂CH(OH)CH₂OR₁, or
R, if n is 2, is a radical of formula -CH₂CH(OH)CH₂-O-R₁₂-O-CH₂CH(OH)CH₂- or -(CH₂)ₘCO-O-R₁₄-O-CO(CH₂)ₘ-, wherein m is 1, 2 or 3, and R₁₂ is alkylene of 2 to 10 carbon atoms, alkylene of 3 to 14 carbon atoms which is interrupted by oxygen, or is phenylene or a radical of formula 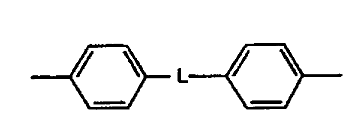 wherein L is -O-, -S-, -SO₂-, -CH₂- or -C(CH₃)₂-, and
R₁₄ is alkylene of 2 to 10 carbon atoms, or alkylene of 4 to 30 carbon atoms which is interrupted by oxygen, or
R, if n is 3, is a radical of formula 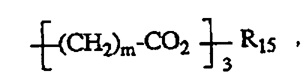 wherein m has the given meaning, and
R₁₅ is alkanetetrayl of 3 to 12 carbon atoms, or
R, if n is 4, is a radical of formula 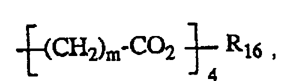 wherein m has the given meaning, and
R₁₆ is alkanetetrayl of 4 to 12 carbon atoms.

2. A compound according to claim 1, wherein
R, if n is 1, is alkyl of 1 to 15 carbon atoms which is substituted by -CO₂R₂ or is -CH₂CH(OH)CH₂OR₁,
and R₁ is hydrogen, alkyl of 1 to 18 carbon atoms, cyclohexyl or phenyl, R₂ is alkyl of 1 to 12 carbon atoms, alkyl or hydroxyalkyl each of 3 to 14 carbon atoms and interrupted by oxygen, hydroxyalkyl of 2 to 12 carbon atoms, alkenyl of 3 to 18 carbon atoms, cyclohexyl, glycidyl, benzyl, alkylphenyl containing 1 to 12 carbon atoms in the alkyl moiety, phenyl or furfuryl, or
R, if n is 2, is a radical of formula -CH₂CH(OH)CH₂-O-R₁₂-O-CH₂CH(OH)CH₂- or -(CH₂)ₘCO-O-R₁₄-O-CO(CH₂)ₘ-, wherein m is 1 or 2,
and R₁₂ is alkylene of 2 to 10 carbon atoms, and
R₁₄ is alkylene of 2 to 10 carbon atoms, or alkylene of 4 to 20 carbon atoms which is interrupted by oxygen.

3. A compound according to claim 1, wherein
R, if n is 1, is alkyl of 1 to 3 carbon atoms which is substituted by -CO₂R₂ or is -CH₂CH(OH)CH₂OR₁,
and R₁ is hydrogen, alkyl of 1 to 18 carbon atoms, cyclohexyl or phenyl, R₂ is alkyl of 1 to 8 carbon atoms, alkyl of 3 to 12 carbon atoms which is interrupted by oxygen, or is vinyl, allyl, cyclohexyl, benzyl or glycidyl, and
R, if n is 2, is a radical of formula -(CH₂)₂CO-O-R₁₄-O-CO(CH₂)₂-, and R₁₄ is alkylene of 2 to 4 carbon atoms, or alkylene of 4 to 12 carbon atoms which is interrupted by oxygen.

4. A compound according to claim 3, wherein n is 1 and R is a radical of formula -CH₂CH(OH)CH₂OR₁, wherein R₁ is hydrogen or alkyl of 1 to 18 carbon atoms, cyclohexyl or phenyl.

5. A compound according to claim 1, wherein R, if n is 2, is a radical of formula 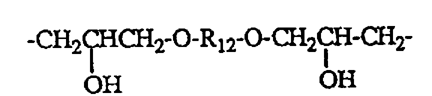 where R₁₂ is as defined in claim 1.

6. A process for the preparation of a compound of formula (1) according to claim 1, which comprises reacting the compound of formula 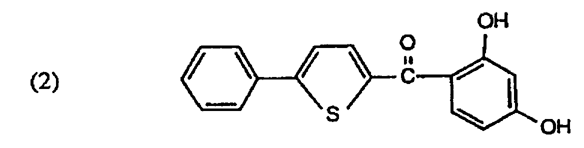 with a halogen compound of formula
(3) R-X,
wherein R is as defined in claim 1 and X is halogen, preferably chloro or bromo.

7. A process for the preparation of a compound of formula (1) according to claim 4, which comprises reacting the compound of formula 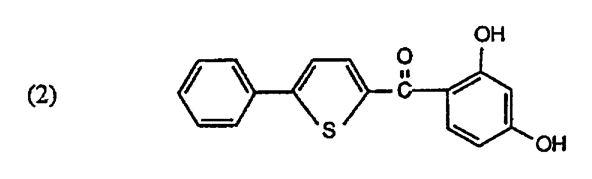 with an epoxide compound of formula 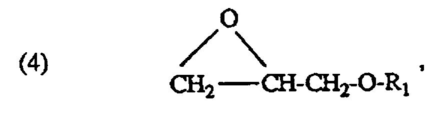 wherein R₁ is as defined in claim 4.

8. A process for the preparation of a compound of formula (1) according to claim 5, which comprises reacting the compound of formula 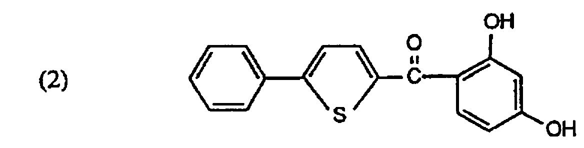 with an epoxide compound of formula 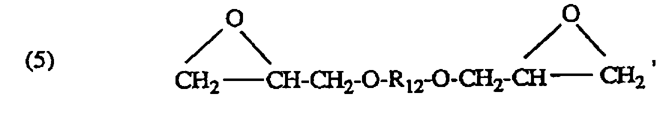 wherein R₁₂ is as defined in claim 5.

9. Photographic material containing one or more silver halide emulsion layers on a transparent support, wherein said material contains an additional layer in which at least one compound of formula (1) is present.

10. Photographic material according to claim 9, which contains the additional layer above the topmost silver halide emulsion layer, between two silver halide emulsion layers or on the back of the support.

## Revendications

1. Composé de formule 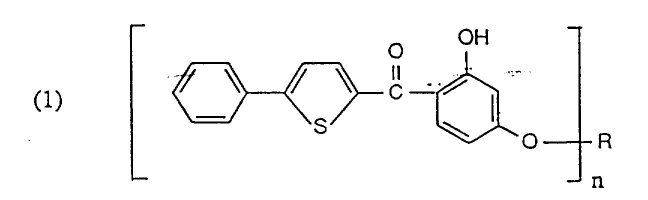 où n est 1, 2, 3 ou 4, et
R, si n est 1, représente un alkyle ayant de 1 à 18 atomes de carbone substitué par -CO₂R₂ ou R représente -CH₂CH(OH)CH₂OR₁, où
R₁ représente l'hydrogène, un alkyle ayant de 1 à 18 atomes de carbone, le cyclohexyle, le benzyle, le phényle, le tolyle, un -CO-alcényle ayant de 2 à 4 atomes de carbone dans la partie alcényle, ou un glycidyloxyalkyle ayant de 3 à 14 atomes de carbone dans la partie alkyle,
R₂ représente un alkyle ayant de 1 à 18 atomes de carbone, un alkyle ou un hydroxyalkyle interrompu par l'oxygène ayant chaque fois de 3 à 14 atomes de carbone, un hydroxyalkyle ayant de 2 à 18 atomes de carbone, un glycidyle, un cycloalkyle ayant de 5 à 12 atomes de carbone, un alcényle ayant de 3 à 18 atomes de carbone, le benzyle, le phényle, un alkylphényle ayant de 1 à 12 atomes de carbone dans la partie alkyle, le furfuryle ou un radical de formule -CH₂CH(OH)CH₂OR_{1,} ou
R, si n est 2, représente un radical de formule -CH₂CH(OH)CH₂-O-R₁₂-O-CH₂CH(OH)CH₂- ou -(CH₂)ₘCO-O-R₁₄-O-CO(CH₂)ₘ-, où m est 1, 2 ou 3, où
R₁₂ est un alkylène ayant de 2 à 10 atomes de carbone, un alkylène interrompu par l'oxygène ayant de 3 à 14 atomes de carbone, le phénylène ou un radical de formule 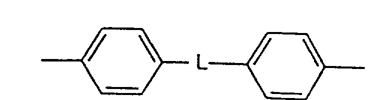
où L est -O-, -S-, -SO₂-, -CH₂- ou -C(CH₃)₂-, et
R₁₄ représente un alkylène ayant de 2 à 10 atomes de carbone ou un alkylène interrompu par l'oxygène ayant de 4 à 30 atomes de carbone, ou
R, si n est 3, représente un radical de formule -[-(CH₂)ₘ-CO₂-]₃-R₁₅, où m a la signification donnée, et
R₁₅ est un alcanetriyle, ayant de 3 à 12 atomes de carbone, ou
R, si n est 4, représente un radical de formule -[-(CH₂)m-CO₂-]₄-R₁₆, où m a la signification donnée, et
R₁₆ est un alcanetétrayle ayant de 4 à 12 atomes de carbone.

2. Composés selon la revendication 1, où
R, si n est 1, représente un alkyle ayant de 1 à 15 atomes de carbone, substitué par -CO₂R₂, ou -CH₂CH(OH)CH₂OR₁, où
R₁ représente l'hydrogène, un alkyle ayant de 1 à 18 atomes de carbone, le cyclohexyle ou le phényle,
R₂ représente un alkyle ayant de 1 à 12 atomes de carbone, un alkyle ou hydroxyalkyle interrompu par l'oxygène ayant chaque fois de 3 à 14 atomes de carbone, un hydroxyalkyle ayant de 2 à 12 atomes de carbone, un alcényle ayant de 3 à 18 atomes de carbone, le cyclohexyle, le glycidyle, le benzyle, un alkylphényle ayant de 1 à 12 atomes de carbone dans la partie alkyle, le phényle ou le furfuryle,
R, si n est 2, représente un radical de formule -CH₂CH(OH)CH₂-O-R₁₂-O-CH₂CH(OH)CH₂- ou -(CH₂)ₘCO-O-R₁₄-O-CO(CH₂)m-, où m est 1, ou 2, où
R₁₂ est un alkylène ayant de 2 à 10 atomes de carbone, et
R₁₄ est un alkylène ayant de 2 à 10 atomes de carbone ou un alkylène interrompu par l'oxygène ayant de 4 à 20 atomes de carbone.

3. Composés selon la revendication 2, où
R, si n est 1, représente un alkyle ayant de 1 à 3 atomes de carbone, substitué par -CO₂R₂, ou -CH₂CH(OH)CH₂OR₁, où
R₁ représente l'hydrogène, un alkyle ayant de 1 à 18 atomes de carbone, le cyclohexyle ou le phényle,
R₂ représente un alkyle ayant de 1 à 8 atomes de carbone, un alkyle interrompu par l'oxygène ayant de 3 à 12 atomes de carbone, le vinyle, l'allyle, le cyclohexyle, le benzyle ou le glycidyle, et
R, si n est 2, représente un radical de formule -(CH₂)₂CO-O-R₁₄-O-CO (CH₂)₂-, où
R₁₄ est un alkylène ayant de 2 à 4 atomes de carbone ou un alkylène interrompu par l'oxygène ayant de 4 à 12 atomes de carbone.

4. Composés selon la revendication 3, où
n est 1, et R est un radical de formule -CH₂CH(OH)CH₂OR_{1,} où R₁ est l'hydrogène ou un alkyle ayant de 1 à 18 atomes de carbone, le cyclohexyle ou le phényle.

5. Composés selon la revendication 1, où
R, si n est 2, représente un radical de formule 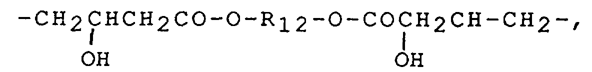 où R₁₂ a la signification donnée dans la revendication 1.

6. Procédé pour préparer les composés de formule (1) selon la revendication 1, caractérisé en ce que l'on fait réagir le composé de formule (2) 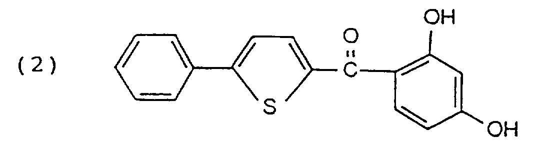 avec des composés halogénés de formule (3)
R-X,
où R a la signification donnée dans la revendication 1 et X représente un halogène, de préférence le chlore ou le brome.

7. Procédé pour la préparation des composés de formule (1) selon la revendication 4, caractérisé en ce que l'on fait réagir le composé de formule 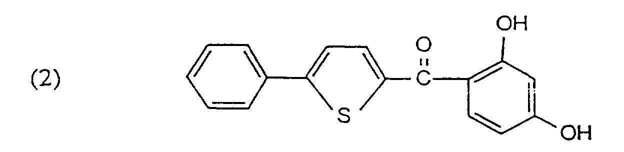 avec un composé époxyde de formule (4) 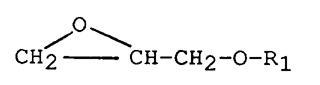 où R₁ a la signification donnée dans la revendication 4.

8. Procédé pour la préparation des composés de formule (1) selon la revendication 5, caractérisé en ce que l'on fait réagir le composé de formule 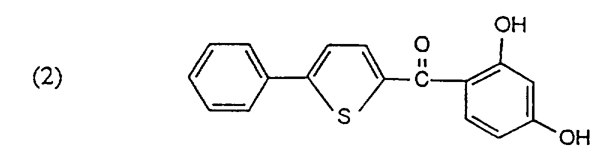 avec un composé époxyde de formule 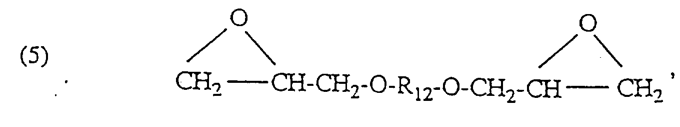 où R₁₂ a la signification donnée dans la revendication 5.

9. Matière photographique comportant une ou plusieurs couches d'émulsion à halogénure d'argent sur un support transparent, caractérisée en ce qu'elle comporte comme couche additionnelle une couche qui comporte au moins un composé de formule (1).

10. Matière photographique selon la revendication 9, caractérisée en ce qu'elle comporte la couche additionnelle sur la couche supérieure à émulsion d'halogénure d'argent, entre deux couches à émulsion d'halogénure d'argent ou sur la face arrière du support.
